Europäisches Patentamt

European Patent Office

Office européen des brevets

(19)

(11) Numéro de publication : **0 355 907 B1**

# (12) FASCICULE DE BREVET EUROPEEN

(45) Date de publication du fascicule du brevet :
**08.07.92 Bulletin 92/28**

(21) Numéro de dépôt : **89202065.2**

(22) Date de dépôt : **11.08.89**

(51) Int. Cl.⁵ : $B01J\ 23/58$, $B01J\ 23/78$,
$B01J\ 27/128$, $C07C\ 21/18$,
$C07C\ 17/34$

(54) **Procédé d'hydrogénation de 1,1,2-trichloro-1,2,2-trifluoréthane.**

(30) Priorité : **22.08.88 FR 8811152**

(43) Date de publication de la demande :
**28.02.90 Bulletin 90/09**

(45) Mention de la délivrance du brevet :
**08.07.92 Bulletin 92/28**

(84) Etats contractants désignés :
**AT BE CH DE ES FR GB GR IT LI LU NL SE**

(56) Documents cités :
**EP-A- 0 053 657**
**EP-A- 0 181 026**
**EP-A- 0 343 707**
**GB-A- 931 643**
**US-A- 2 685 606**
**US-A- 2 864 873**
**US-A- 3 846 281**

(73) Titulaire : **SOLVAY**
**Rue du Prince Albert, 33**
**B-1050 Bruxelles (BE)**

(72) Inventeur : **Lerot, Luc**
**Avenue du Centaure 31**
**B-1200 Bruxelles (BE)**
Inventeur : **Wilmet, Vincent**
**Rue Cour Boisacq 81**
**B-1301 Bierges (BE)**
Inventeur : **Pirotton, Joseph**
**Rue Désiré Desmet 28**
**B-1030 Bruxelles (BE)**

(74) Mandataire : **Nichels, William et al**
**Solvay Département de la Propriété**
**Industrielle Rue de Ransbeek, 310**
**B-1120 Bruxelles (BE)**

EP 0 355 907 B1

## Description

L'invention concerne un procédé d'hydrogénation de 1,1,2-trichloro-1,2,2-trifluoréthane en chlorotrifluoréthylène et trifluoréthylène.

L'hydrogénation de chlorofluoréthanes à l'intervention de catalyseurs comprenant d'une part un support tel que l'alumine et d'autre part un métal du groupe VIII de la table périodique des éléments est une réaction connue depuis longtemps (brevet US 2697124).

Ces catalyseurs ont subi des améliorations multiples et ont conduit à des procédés tels que notamment décrits dans le brevet européen 53657 qui concerne notamment un procédé d'hydrogénation de 1,1,2-trichloro-1,2,2-trifluoréthane en chlorotrifluoréthylène ou en trifluoréthylène à l'intervention de catalyseurs constitués d'un métal du groupe du platine déposé sur un support particulier tel qu'un sel mixte de fluorure de sodium magnésium ou de fluorure de potassium magnésium. De tels catalyseurs présentent l'avantage de pouvoir être réactivés à température élevée, telle que 400 à 600°C, par un gaz contenant de l'oxygène.

Toutefois, tous les procédés de synthèse catalytique connus à ce jour ont une activité catalytique relativement faible et sont accompagnés de réactions secondaires et/ou une désactivation rapide des catalyseurs, ce qui compromet l'efficacité de ces procédés.

L'invention, par contre, concerne un procédé de synthèse catalytique qui ne présente plus ces inconvénients. On a en effet trouvé des compositions catalytiques qui permettent d'hydrogéner le 1,1,2-trichloro-1,2,2-trifluoréthane avec une sélectivité et un taux de transformation, c'est-à-dire un rendement, qui n'ont jamais été atteints industriellement, qui présentent l'avantage d'être stables et de se désactiver beaucoup plus lentement que les compositions catalytiques connues et qui en outre sont régénérables à température modérée.

L'invention concerne à cet effet un procédé d'hydrogénation du 1,1,2-trichloro-1,2,2-trifluoréthane en chlorotrifluoréthylène et trifluoréthylène en présence d'hydrogène moléculaire, caractérisé en ce que la réaction est catalysée par une composition catalytique comprenant un support poreux oxygéné ou à base de carbone sur lequel sont déposés un métal du groupe VIII de la table périodique des éléments et un ou plusieurs composés choisis parmi les sels d'un métal alcalin ou alcalino-terreux.

Les sels d'un métal alcalin ou alcalino-terreux utilisés sont choisis parmi les sels organiques ou inorganiques de ces métaux. Comme sels organiques, on utilise généralement des carboxylates, des alcoolates et des acétylacétonates, dont la chaîne alkyle contient habituellement de 1 à 10 atomes de carbone. Comme sels inorganiques, on utilise généralement des halogénures, des hydroxydes ou des nitrates et, plus particulièrement, les halogénures ou les hydroxydes d'un métal alcalin ou alcalino-terreux, tels que les chlorures, fluorures ou hydroxydes de sodium, potassium, césium, lithium, baryum, calcium et rubidium. De manière avantageuse, on choisit les chlorures, fluorures ou hydroxydes de sodium, potassium, césium ou baryum, tels que le chlorure de césium, le chlorure de potassium, le chlorure de baryum, le fluorure de césium et l'hydroxyde de césium. De manière préférée, on utilise le chlorure de césium, le chlorure de potassium ou le chlorure de baryum.

Les compositions catalytiques peuvent comprendre un ou plusieurs composés choisis parmi les sels d'un métal alcalin ou alcalino-terreux. De bons résultats ont été obtenus avec un ou deux de ces composés. On utilise de préférence une composition binaire choisie parmi les chlorures de césium, potassium ou baryum. Particulièrement préférées sont les compositions contenant simultanément du chlorure de baryum et du chlorure de césium.

Les compositions catalytiques selon l'invention comprennent généralement de 1 à 25 % en poids de métal alcalin ou alcalino-terreux par rapport au poids total de la composition catalytique. De préférence, elles comprennent de 5 à 20 % en poids de métal alcalin ou alcalino-terreux par rapport au poids total de la composition catalytique.

Lorsqu'on utilise des compositions catalytiques constituées de plusieurs composés, les proportions entre chaque composé peuvent varier entre de larges limites. De bons résultats ont été obtenus avec le chlorure de baryum et le chlorure de césium mis en oeuvre dans des rapports en baryum et césium compris entre 2:1 et 1:2 en poids.

Comme métal du groupe VIII de la table périodique des éléments mis en oeuvre dans les compositions catalytiques de l'invention, on utilise habituellement le palladium, le platine, le rhodium, le ruthénium, le cobalt ou le nickel et, de préférence, le palladium ou le platine.

Les compositions catalytiques selon l'invention comprennent habituellement de 0,05 à 10 % en poids de métal du groupe VIII par rapport au poids total de la composition catalytique et de préférence de 0,1 à 5 %.

De bons résultats ont été obtenus lorsque le rapport pondéral entre le métal alcalin ou alcalino-terreux et le métal du groupe VIII est compris entre 0,1 et 15 et, plus particulièrement lorsque ce rapport est compris entre 2 et 6.

Habituellement, on utilise comme support des compositions catalytiques de l'invention un support poreux à base de carbone tel que le charbon actif ou un support poreux oxygéné à base d'alumine, de silice, de titane, de magnésium ou de zirconium. De bons résultats ont été obtenus avec l'alumine, la silice et les mélanges d'alumine et de silice, ainsi qu'avec l'oxyde de titane et l'oxyde de zirconium.

Le volume poreux du support mis en oeuvre peut varier entre de larges limites et est généralement compris entre 0,1 et 5 cm$^3$/g et habituellement entre 0,3 et 2 cm$^3$/g.

La surface spécifique du support est généralement comprise entre 5 et 1000 m$^2$/g, et habituellement entre 10 et 750 m$^2$/g.

Les compositions catalytiques peuvent être obtenues par imprégnation du support avec des solutions contenant le métal du groupe VIII de la table périodique des éléments et un ou plusieurs composés choisis parmi les sels d'un métal alcalin ou alcalino-terreux. Cette imprégnation peut être réalisée par n'importe quelle méthode, telle que notamment par la technique dite du "volume poreux" (imprégnation dite "sèche") ou par la technique du "volume excédentaire" (imprégnation par voie dite "humide"); ces méthodes sont décrites dans la littérature et plus particulièrement par Charles N. Satterfield "Heterogeneous catalysis in practice", 1980, Mc Graw-Hill Book Company, New York, en particulier pages 82 et 83.

Le métal du groupe VIII de la table périodique des éléments est habituellement introduit dans les compositions de l'invention sous la forme d'un sel de ce métal. Pour ce faire, on met généralement en oeuvre un chlorure ou un complexe ammoniacal du métal du groupe VIII.

Les solutions d'imprégnation peuvent être aqueuses ou organiques, de préférence on met en oeuvre une solution aqueuse ou alcoolique.

L'imprégnation peut être réalisée d'abord avec une solution contenant le métal du groupe VIII de la table périodique des éléments, ou d'abord avec une solution contenant un ou plusieurs composés choisis parmi les sels d'un métal alcalin ou alcalino-terreux, ou simultanément avec les deux solutions.

Un mode d'obtention des compositions catalytiques de l'invention ayant donné de bons résultats consiste à imprégner le support lors d'une première étape avec une solution aqueuse contenant un ou plusieurs composés choisis parmi les sels d'un métal alcalin ou alcalino-terreux, puis, après séchage, lors d'une deuxième étape avec une solution aqueuse contenant un sel du métal du groupe VIII de la table périodique des éléments, sel soluble dans l'eau, tel que notamment un chlorure. Ces imprégnations se font généralement à température ambiante avec une solution aqueuse renfermant les quantités désirées de sels d'un métal alcalin ou alcalino-terreux, puis de sel du métal du groupe VIII. Le séchage entre les deux imprégnations a lieu à 350°C pendant 2 heures. Le support imprégné est ensuite séché à 120°C, puis introduit dans le réacteur d'hydrogénation proprement dit. La composition catalytique ainsi obtenue peut être mise en oeuvre telle quelle ou peut être préalablement réduite soit par de l'hydrogène, soit par un mélange d'hydrogène avec un gaz inerte tel que l'hélium. La température à laquelle est effectuée cette réduction est généralement comprise entre 100 et 500°C; de bons résultats ont été obtenus avec une température de réduction comprise entre 150 et 250°C. La pression à laquelle est effectuée cette réduction est généralement comprise entre 1 et 5 bars.

Les compositions catalytiques selon l'invention peuvent être mises en oeuvre dans tout procédé d'hydrogénation, tel que notamment les procédés réalisés avec un catalyseur disposé en lit fixe ou en lit fluidisé.

La température à laquelle s'effectue la réaction d'hydrogénation se situe habituellement entre 80 et 600°C. De préférence, cette température est comprise entre 120 et 400°C. De bons résultats ont été obtenus avec une température réactionnelle située aux environs de 200-300°C.

La pression à laquelle est effectuée la réaction d'hydrogénation n'est pas critique en elle-même. Habituellement, on opère avec des pressions comprises entre 1 et 10 bars et, de préférence, avec des pressions comprises entre 2 et 5 bars.

Le rapport volumique entre le 1,1,2-trichloro-1,2,2-trifluoréthane et l'hydrogène mis en oeuvre est généralement compris entre 0,05 et 4. De préférence, ce rapport est compris entre 0,1 et 2,5. De bons résultats ont été obtenus avec un rapport situé aux environs de 1.

Le temps de contact moyen est généralement compris entre 2 et 16 s; habituellement ce temps est compris entre 3 et 10 s. De bons résultats ont été obtenus avec un temps de contact compris entre 4 et 8 s.

Le procédé d'hydrogénation peut être réalisé en présence d'un gaz inerte tel que l'hélium.

Les compositions catalytiques selon l'invention permettent d'obtenir un taux de transformation de 1,1,2-trichloro-1,2,2-trifluoréthane élevé, supérieur à 40 %.

Le rapport du chlorotrifluoréthylène et du trifluoréthylène dans le mélange réactionnel à la sortie du réacteur peut être modulé dans une large plage suivant les conditions de réaction avec les compositions catalytiques selon l'invention.

Après utilisation des compositions catalytiques de l'invention, on observe que la régénération de la composition catalytique est aisée et peut se réaliser in situ dans le réacteur d'hydrogénation. Un mode de régénération ayant donné de bons résultats consiste à régénérer les compositions catalytiques sous courant d'air, puis sous courant d'hydrogène. Les performances des compositions catalytiques après la régénération sont très proches de celles observées avec des compositions catalytiques fraîches. Cette régénération des compositions catalytiques est généralement réalisée à température modérée, c'est-à-dire à des températures comprises entre 100 et 600°C et de préférence 200 et 400°C.

L'invention se trouve plus amplement illustrée par les exemples suivants.

Exemple 1 :

a) Préparation de la composition catalytique

On introduit dans une ampoule à imprégnation cylindrique de 40 cm³ 10 g de silice ayant les caractéristiques suivantes :
– surface spécifique B.E.T. de 250 m²/g
– volume poreux environ 0,8 cm³/g.

On chauffe l'ampoule sous vide (3 mm Hg) durant 2 heures à 350°C dans un four cylindrique en vue de dégazer et sécher la silice.

Après refroidissement sous vide, on imprègne la silice à température ambiante sous vide dans un volume de 8 cm³ d'une solution aqueuse contenant 1 g de chlorure de césium et 1,30 g de chlorure de baryum.

On laisse reposer 1 heure sous vide statique, puis une nuit à pression atmosphérique à température ambiante.

Ensuite, on sèche la silice ainsi imprégnée à 350°C sous vide (3 mm Hg) durant 2 heures.

On imprègne ensuite cette silice à température ambiante sous vide (3 mm Hg) dans un volume de 8 cm³ d'une solution contenant 0,14 g de chlorure de palladium dans de l'eau acidifiée par 4 % en volume d'acide chlorhydrique concentré.

On laisse reposer 1 heure sous vide, puis une nuit à pression atmosphérique à température ambiante.

Puis, on sèche 3 heures à 120°C à pression atmosphérique.

La composition catalytique ainsi obtenue comprend 0,67 % en poids de palladium, 6,8 % en poids de baryum et 6,4 % en poids de césium par rapport au poids total de la composition catalytique.

2 cm³ de cette composition catalytique sont introduits dans un réacteur d'hydrogénation constitué d'un tube métallique en inox long de 520 mm et d'un diamètre intérieur de 7,7 mm; puis, la composition catalytique est conditionnée 2 heures à 500°C sous 3 bars au moyen d'un mélange d'hydrogène et d'hélium dans un rapport volumique 1/9 à un débit de 40 cm³/mn.

b) Hydrogénation du 1,1,2-trichloro-1,2,2-trifluoréthane

On alimente le réacteur à raison de 0,01 mole par heure de 1,1,2-trichloro-1,2,2-trifluoréthane, 0,015 mole par heure d'hydrogène et 0,08 mole par heure d'hélium à 240°C sous 3 bars. Le temps de contact moyen est évalué à 4,8 s.

Après 10 heures de fonctionnement, le taux de transformation du 1,1,2-trichloro-1,2,2-trifluoréthane est de 55 % (volumique), la sélectivité est de 57 % en trifluoréthylène et de 20 % en chlorotrifluoréthylène.

Exemple 2 :

Une composition catalytique est préparée en suivant le protocole décrit à l'exemple 1a.

Le support est constitué de silice telle que celle décrite à l'exemple 1.

On met en oeuvre 10 % en poids de chlorure de baryum, 7,7 % en poids de chlorure de césium et 4,6 % en poids de chlorure de palladium calculé par rapport au poids total de la composition catalytique.

La composition catalytique est réduite dans le réacteur, identique à celui décrit à l'exemple 1, pendant 2 heures à 500°C sous 3 bars au moyen d'un mélange d'hydrogène et d'hélium dans un rapport volumique 1/9 à un débit de 40 cm³/mn.

On alimente le réacteur à raison de 0,043 mole par heure de 1,1,2-trichloro-1,2,2-trifluoréthane et 0,064 mole par heure d'hydrogène à 240°C sous 3 bars. Le temps de contact moyen est évalué à 4,8 s.

Après 4 heures de fonctionnement, le taux de transformation du 1,1,2-trichloro-1,2,2-trifluoréthane est de 54 %, la sélectivité est de 55 % en trifluoréthylène et de 22 % en chlorotrifluoréthylène.

Exemple 3 :

La composition catalytique mise en oeuvre est identique à celle décrite à l'exemple 2.

On alimente le réacteur à raison de 0,043 mole par heure de 1,1,2-trichloro-1,2,2-trifluoréthane et 0,064 mole par heure d'hydrogène à 260°C sous 3 bars.

Le temps de contact moyen est évalué à 4,6 s.

Après 4 heures de fonctionnement, le taux de transformation du 1,1,2-trichloro-1,2,2-trifluoréthane est de 48 %, la sélectivité est de 30 % en chlorotrifluoréthylène et de 51 % en trifluoréthylène.

Exemple 4 :

Une composition catalytique mise en oeuvre est identique à celle décrite à l'exemple 2.

On alimente le réacteur à raison de 0,043 mole par heure de 1,1,2-trichloro-1,2,2-trifluoréthane et 0,064 mole par heure d'hydrogène à 200°C sous 3 bars. Le temps de contact moyen est évalué à 5,2 s.

Après 4 heures de fonctionnement, le taux de transformation du 1,1,2-trichloro-1,2,2-trifluoréthane est de 37 %, la sélectivité est de 53 % en trifluoréthylène et de 15 % en chlorotrifluoréthylène.

Après 70 heures de fonctionnement, le taux de transformation du 1,1,2-trichloro-1,2,2-trifluoréthane est de 27 %, la sélectivité est de 53 % en trifluoréthylène et de 25 % en chlorotrifluoréthylène.

Exemples 5, 6, 7, 8 et 9

Une composition catalytique telle que décrite à l'exemple 2 est mise en oeuvre.

Les conditions de mise en oeuvre et les résultats après 10 heures de fonctionnement sont rassemblés dans le tableau 1.

Exemple 10

Une composition catalytique est préparée en suivant le protocole décrit à l'exemple 1a.

Le support est constitué d'oxyde de titane ayant les caractéristiques suivantes :

– surface spécifique : 120 m$^2$/g

– volume poreux : 0,4 cm$^3$/g.

On met en oeuvre 7,8 % en poids de chlorure de césium, 10 % en poids de chlorure de baryum et 4 % en poids de chlorure de palladium calculé par rapport au poids total de la composition catalytique.

La composition catalytique est réduite dans un réacteur, identique à celui décrit à l'exemple 1, pendant 2 heures à 260°C.

On alimente le réacteur à raison de 0,010 mole par heure de 1,1,2-trichloro-1,2,2-trifluoréthane, 0,015 mole par heure d'hydrogène et 0,080 mole par heure d'hélium à 260°C sous 3 bars. Le temps de contact moyen est évalué à 4,6 s.

Après 4 heures de fonctionnement, le taux de transformation du 1,1,2-trichloro-1,2,2-trifluoréthane s'élève à 62 %.

Après 20 heures de fonctionnement, on procède à une régénération.

On régénère la composition catalytique in situ dans le réacteur d'hydrogénation.

Pour ce faire, on introduit dans le réacteur un courant d'air durant 2 heures à 450°C, puis un courant d'un mélange d'hydrogène et d'hélium dans un rapport volumique 1/9 à un débit de 40 cm$^3$/mn à 260°C durant 2 heures.

Ensuite, on alimente à nouveau le réacteur dans les conditions décrites précédemment.

Après 25 heures de fonctionnement total, le taux de transformation du 1,1,2-trichloro-1,2,2-trifluoréthane s'élève à 62 %, après 60 heures de fonctionnement total, il est de 25 %.

On procède de nouveau à une régénération à ce stade, c'est-à-dire après 60 heures de fonctionnement total, dans les conditions décrites précédemment.

Ensuite, on alimente de nouveau le réacteur.

Après 65 heures de fonctionnement total, le taux de transformation du 1,1,2-trichloro-1,2,2-trifluoréthane s'élève à 60 %, après 85 heures de fonctionnement total, il est de 42 %.

Tableau 1

EP 0 355 907 B1

| EXEMPLES | ALIMENTATION DU REACTEUR EN MOLE PAR HEURE | | | TEMPERA-TURE °C | TEMPS DE SEJOUR s | TAUX DE TRANS-FORMATION DU * % VOLUMIQUE | SELECTIVITE EN | |
|---|---|---|---|---|---|---|---|---|
| | * | HYDRO-GENE | HELIUM | | | | *** % VOLUMIQUE | ** % VOLUMIQUE |
| 5 | 0,010 | 0,015 | 0,08 | 240 | 4,8 | 60 | 21 | 63 |
| 6 | 0,020 | 0,030 | 0,054 | 240 | 4,8 | 61 | 19 | 64 |
| 7 | 0,040 | 0,060 | 0 | 240 | 4,8 | 54 | 21 | 55 |
| 8 | 0,040 | 0,060 | 0,107 | 240 | 1,2 | 45 | 27 | 52 |
| 9 | 0,0215 | 0,043 | 0,043 | 240 | 4,8 | 61 | 19 | 59 |

\*    : 1,1,2-trichloro-1,2,2-trifluoréthane

\*\*   : trifluoréthylène

\*\*\* : chlorotrifluoréthylène.

Exemple 11 :

a) Préparation de la composition catalytique

On introduit dans une ampoule à imprégnation cylindrique de 40 cm³ 23,03 g d'oxyde de magnésium ayant les caractéristiques suivantes :
– surface spécifique B.E.T. de 23 m²/g
– volume poreux environ 0,3 cm³/g,
et préalablement imbibés par 6,3 cm³ d'eau, laissés au repos 16 heures puis séchés sous vide (3 mm Hg) durant 2 heures.

On imprègne l'oxyde de magnésium à température ambiante sous vide dans un volume de 10 cm³ d'une solution aqueuse contenant 1,25 g de chlorure de césium.

On laisse reposer 1 heure sous vide statique.

Ensuite, on sèche l'oxyde de magnésium à 125°C sous vide (3 mm Hg) durant 1 heure.

On imprègne ensuite cet oxyde de magnésium à température ambiante sous vide (3 mm Hg) dans un volume de 8 cm³ d'une solution contenant 0,212 g de chlorure de palladium dans de l'eau acidifiée par 10 % en volume d'acide chlorhydrique concentré.

On laisse reposer 1 heure sous vide.

Puis, on sèche 1 heure à 125°C sous vide.

La composition catalytique ainsi obtenue comprend 0,86 % en poids de palladium et 4 % en poids de césium par rapport au poids total de la composition catalytique.

2 cm³ de cette composition catalytique sont introduits dans un réacteur d'hydrogénation constitué d'un tube métallique en inox long de 520 mm et d'un diamètre intérieur de 7,7 mm; puis, la composition catalytique est conditionnée 2 heures à 240°C sous 3 bars au moyen d'un mélange d'hydrogène et d'hélium dans un rapport volumique 1/9 à un débit de 40 cm³/mn.

b) Hydrogénation du 1,1,2-trichloro-1,2,2-trifluoréthane

On alimente le réacteur à raison de 0,011 mole par heure de 1,1,2-trichloro-1,2,2-trifluoréthane, 0,016 mole par heure d'hydrogène et 0,08 mole par heure d'hélium à 240°C sous 3 bars. Le temps de contact moyen est évalué à 4,8 s.

Après 10 heures de fonctionnement, le taux de transformation du 1,1,2-trichloro-1,2,2-trifluoréthane est de 70 % (molaire), la sélectivité est de 60 % en trifluoréthylène et de 12 % en chlorotrifluoréthylène.

**Revendications**

1. Procédé d'hydrogénation de 1,1,2-trichloro-1,2,2-trifluoréthane en chlorotrifluoréthylène et trifluoréthylène en présence d'hydrogène moléculaire, caractérisé en ce que la réaction est catalysée par une composition catalytique comprenant un suppport poreux oxygéné ou à base de carbone sur lequel sont déposés un métal du groupe VIII de la table périodique des éléments et un ou plusieurs composés choisis parmi les sels d'un métal alcalin ou alcalino-terreux.

2. Procédé selon la revendication 1, caractérisé en ce que les sels d'un métal alcalin ou alcalino-terreux utilisés sont des halogénures ou des hydroxydes de ces métaux.

3. Procédé selon la revendication 2, caractérisé en ce que les halogénures ou les hydroxydes d'un métal alcalin ou alcalino-terreux utilisés sont choisis parmi les chlorures, fluorures ou hydroxydes de potassium, césium ou baryum.

4. Procédé selon la revendication 2 ou 3, caractérisé en ce que la composition catalytique comprend deux composés choisis parmi les halogénures ou les hydroxydes d'un métal alcalin ou alcalino-terreux.

5. Procédé selon l'une quelconque des revendications précédentes, caractérisé en ce que la composition catalytique comprend de 1 à 25 % en poids de métal alcalin ou alcalino-terreux.

6. Procédé selon l'une quelconque des revendications précédentes, caractérisé en ce que le métal du groupe VIII de la table périodique des éléments est choisi parmi le palladium ou le platine.

7. Procédé selon l'une quelconque des revendications précédentes, caractérisé en ce que la composition catalytique comprend de 0,05 à 10 % en poids de métal du groupe VIII.

8. Procédé selon l'une quelconque des revendications précédentes, caractérisé en ce que le support oxygéné utilisé est à base d'alumine, de silice, d'un mélange d'alumine et de silice, d'oxyde de titane ou d'oxyde de zirconium.

**Patentansprüche**

1. Verfahren zur Hydrierung von 1,1,2-Trichloro-1,2,2-trifluoroethan in Chlortrifluorethylen und Trifluorethylen in Anwesenheit von molekularem Wasserstoff, dadurch gekennzeichnet, daß die Reaktion katalysiert wird durch eine katalytische Zusammensetzung, die einen sauerstoffhaltigen porösen Träger oder einen Träger auf Basis von Kohlenstoff umfaßt, auf dem ein Metall der Gruppe VIII der Tabelle des periodischen Systems und eine oder mehrere Verbindungen, ausgewählt unter den Salzen eines Alkali- oder Erdalkalimetalls, abgelagert sind.

2. Verfahren nach Anspruch 1, dadurch gekennzeichnet, daß die verwendeten Salze eines Alkali- oder Erdalkalimetalls Halogenide oder Hydroxyde dieser Metalle sind.

3. Verfahren nach Anspruch 2, dadurch gekennzeichnet, daß die verwendeten Halogenide oder Hydroxyde eines Alkali- oder Erdalkalimetalls ausgewählt sind unter den Chloriden, Fluoriden oder Hydroxyden von Kalium, Cäsium oder Barium.

4. Verfahren nach Anspruch 2 oder 3, dadurch gekennzeichnet, daß die katalytische Zusammensetzung zwei Verbindungen umfaßt, ausgewählt unter den Halogeniden oder Hydroxyden eines Alkali- oder Erdalkalimetalls.

5. Verfahren nach einem der vorhergehenden Ansprüche, dadurch gekennzeichnet, daß die katalytische Zusammensetzung 1 bis 25 Gew.-% Alkali- oder Erdalkalimetall umfaßt.

6. Verfahren nach einem der vorhergenenden Anspruche, dadurch gekennzeichnet, daß das Metall der Gruppe VIII der Tabelle des periodischen Systems ausgewählt ist unter Palladium oder Platin.

7. Verfahren nach einem der vorhergehenden Ansprüche, dadurch gekennzeichnet, daß die katalytische Zusammensetzung 0,05 bis 10 Gew.-% des Metalls der Gruppe VIII umfaßt.

8. Verfahren nach einem der vorhergehenden Ansprüche, dadurch gekennzeichnet, daß der verwendete sauerstoffhaltige Träger auf der Basis von Aluminiumoxyd, Kieselerde, einer Mischung von Aluminiumoxyd und Kieselerde, Titanoxyd oder Zirkoniumoxyd ist.

**Claims**

1. Process for hydrogenation of 1,1,2-trichloro-1,2,2-trifluoroethane to chlorotrifluoroethylene and trifluoroethylene in the presence of molecular hydrogen, characterized in that the reaction is catalyzed by a catalytic composition comprising a porous oxygenated or carbon-based support on which are deposited a metal of group VIII of the periodic table of the elements, and one or more compounds chosen from the salts of an alkali or alkaline-earth metal.

2. Process according to Claim 1, characterized in that the salts of an alkali or alkaline-earth metal used are halides or hydroxides of these metals.

3. Process according to Claim 2, characterized in that the halides or the hydroxides of an alkali or alkaline-earth metal used are chosen from the chlorides, fluorides or hydroxides of potassium, caesium or barium.

4. Process according to Claim 2 or 3, characterized in that the catalytic composition contains two compounds chosen from the halides or the hydroxides of an alkali or alkaline-earth metal.

5. Process according to any one of the preceding claims, characterized in that the catalytic composition contains from 1 to 25 % by weight of alkali or alkaline-earth metal.

6. Process according to any one of the preceding claims, characterized in that the metal of group VIII of the periodic table of the elements is chosen from palladium or platinum.

7. Process according to any one of the preceding claims, characterized in that the catalytic composition contains from 0.05 to 10 % by weight of group VIII metal.

8. Process according to any one of the preceding claims, characterized in that the oxygenated support used is based on alumina, silica, a mixture of alumina and silica, titanium oxide or zirconium oxide.